# FASCICULE DE BREVET EUROPEEN

(11) **EP 4 198 486 B1**
(45) Date de publication et mention de la délivrance du brevet: **10.07.2024**
(21) Numéro de dépôt: 22202625.4
(22) Date de dépôt: 19.10.2022
(51) Int. Cl.: G01N 1/28, B28B 23/00, G01N 1/36, G01N 33/38, B28B 1/093, B28B 23/02

(54) **PROCÉDÉ DE PRODUCTION D'UN ÉCHANTILLON DE BÉTON FISSURÉ PAR FISSURATION CONTRÔLÉE ET DISPOSITIF ASSOCIÉ**
VERFAHREN ZUR HERSTELLUNG EINER PROBE AUS BETON MIT KONTROLLIERTER RISSBILDUNG UND VORRICHTUNG DAFÜR
METHOD FOR PRODUCING CONCRETE SAMPLE CRACKED BY CONTROLLED CRACKING AND RELATED DEVICE

(30) Priorité: 15.12.2021 FR 2113573
(43) Date de publication de la demande: 21.06.2023
(73) Titulaire: CENTRE SCIENTIFIQUE ET TECHNIQUE DU BATIMENT, 77420 Champs-Sur-Marne (FR)
(72) Inventeur: GUILLET, Thierry, 77420 Champs-sur-Marne (FR); BRUCHET, Etienne, 77420 Champs-sur-Marne (FR); AL MANSOURI, Omar, 77420 Champs-sur-Marne (FR); MEGE, Romain, 77420 Champs-sur-Marne (FR); PINOTEAU, Nicolas, 77420 Champs-sur-Marne (FR)
(74) Mandataire: Cabinet Chaillot

(56) Documents cités:
- CN-U- 213 580 318
- JP-B2- 6 841 416
- ELIGEHAUSEN ROLF ET AL: "Testing anchors in cracked concrete - Guidance for testing laboratories: how to generate cracks", CONCRETE INTERNATIONAL, 1 June 2004 (2004-06-01), pages 65 - 71, XP093037672, Retrieved from the Internet <URL:https://www.researchgate.net/publication/270588173_Testing_anchors_in_cracked_concrete_-_Guidance_for_testing_laboratories_how_to_generate_cracks> [retrieved on 20230405]

## Description

La présente invention concerne le domaine technique de la production d'échantillons de béton pour réaliser des essais mécaniques, et porte plus particulièrement sur un procédé et un dispositif pour la production d'un échantillon de béton par fissuration contrôlée permettant de tester des fixations.

Les guides d'essais relatifs aux fixations ancrées dans des corps en béton imposent de tester les fixations dans la situation la plus défavorable, c'est-à-dire lorsque la fixation est ancrée dans un plan de fissuration. Il est donc nécessaire de pouvoir obtenir des échantillons de béton aptes à permettre de réaliser des essais mécaniques sur des fixations disposées dans des plans de fissuration.

La méthode actuelle consiste à disposer des initiateurs de fissures, généralement en rompant l'adhérence sur une portion des armatures longitudinales, qui agissent comme des ressorts postérieurement lors de l'ouverture de la fissure, dans le coffrage d'un échantillon puis, après séchage du béton, à initier une fissuration au droit des initiateurs de fissures. Lesdits initiateurs de fissures permettent de s'assurer de la zone dans laquelle la fissure est créée, mais ne permettent pas de s'assurer d'un plan dans lequel ladite fissure va se propager. Avec cette technique, il est donc nécessaire de vérifier la disposition de la fissure lors de l'implantation de chaque fixation à tester, par exemple à l'aide d'un endoscope pour examiner le perçage dans lequel il est prévu d'implanter la fixation. Par ailleurs, il est très difficile de tester des fixations coulées en place car il n'est pas possible de garantir que ces fixations seront disposées dans un plan de fissuration après fissuration.

Une autre approche est développée dans le brevet japonais JP6841416 B2. Ce document divulgue un dispositif et un procédé permettant de fabriquer un échantillon de béton dans lequel une fissure est simulée. Pour cela, il est prévu de couler un premier bloc de béton, d'attendre le séchage du premier bloc de béton, puis de disposer un film sur la face supérieure de celui-ci afin de couler un second bloc de béton sur le premier bloc. Le film est prévu pour simuler une fissure dans l'échantillon de béton constitué par le premier bloc et le second bloc. Après séchage des deux blocs de béton, un perçage est réalisé entre ceux-ci afin d'implanter une fixation rapportée.

Cependant cette technique présente plusieurs inconvénients, comme par exemple le fait que l'échantillon peut ne pas être homogène en raison du fait que le premier bloc et le second bloc ne sont pas coulés simultanément, du fait que le premier bloc et le second bloc sont coulés l'un sur l'autre et du fait que le film constitue une barrière qui empêche la continuité de l'échantillon entre le premier bloc et le second bloc. Par ailleurs, ce dispositif ne permet pas de produire un échantillon de béton armé et impose l'utilisation d'un bâti externe pour réaliser les essais sur les fixations. CN213580318U et JP6841416B2 présentent des procédés de fabrication d'échantillons de béton selon l'état de la technique.

Par conséquent, les solutions de l'art antérieur proposées pour la production d'un échantillon de béton fissuré présentent toujours des inconvénients et des améliorations sont possibles.

L'invention a notamment pour objectif de proposer un procédé et un dispositif qui permettent d'assurer la propagation de la fissuration dans un plan souhaité dans l'échantillon de béton fabriqué.

Un autre objectif de l'invention est de proposer un procédé et un dispositif qui permettent de garantir que la fissuration est bien réalisée dans le plan de fissuration souhaité, ce qui permet de connaître précisément la position de la fissure sans avoir besoin de réaliser des examens ou contrôles supplémentaires comme c'est le cas lors de l'utilisation d'initiateurs de fissures « classiques ».

Un autre objectif de l'invention est de proposer un procédé et un dispositif qui permettent de produire un échantillon de béton configuré pour tester des fixations coulées en place.

Un autre objectif de l'invention est de proposer un procédé et un dispositif qui permettent de produire un échantillon de béton configuré pour tester des fixations rapportées.

Un autre objectif de l'invention est de proposer un procédé et un dispositif qui permettent de garantir l'homogénéité de l'échantillon de béton et la continuité, avant fissuration, de l'échantillon de part et d'autre du plan de fissuration.

Un autre objectif de l'invention est de proposer un procédé et un dispositif qui permettent de contrôler l'ouverture de fissure dans l'échantillon de béton.

Ainsi, la présente invention a pour objet un procédé de production d'un échantillon de béton fissuré par fissuration contrôlée, caractérisé par le fait qu'il comprend les étapes suivantes : a) mettre en place un coffrage selon des dimensions souhaitées pour l'échantillon, le coffrage comprenant un fond horizontal et au moins trois banches latérales verticales formant avec le fond le coffrage avec une face supérieure ouverte ; b) disposer au moins un cadre de support vertical transversalement au coffrage, l'au moins un cadre de support vertical s'étendant sur tout ou partie de la profondeur du coffrage, un treillis étant tendu sur chaque cadre de support dans le plan de fissuration vertical correspondant, chaque cadre de support vertical définissant un plan de fissuration vertical, chaque plan de fissuration étant perpendiculaire à une direction sélectionnée ; c) couler du béton dans le coffrage par la face supérieure ouverte pour remplir le coffrage, le coulage du béton se faisant de manière équilibrée de chaque côté de chaque treillis de manière à limiter des efforts induits par le coulage du béton dans des directions perpendiculaires aux plans des treillis, le béton comprenant des granulats et une dimension de maille de l'au moins un treillis étant supérieure à une dimension maximale des granulats ; d) homogénéiser le béton dans le coffrage ; e) attendre pendant une période prédéfinie pour le séchage du béton ; f) retirer le coffrage et l'au moins un cadre de support ; et g) initier une fissuration contrôlée dans chaque plan de fissuration souhaité de l'échantillon.

Ce procédé permet de contrôler la propagation de fissures dans l'échantillon de béton produit. En effet, chaque treillis permet de guider une fissure dans un plan respectif, ce qui permet de garantir que la fissure se propage bien selon le plan de fissuration souhaité.

Le coulage du béton lors de l'étape de manière équilibrée de chaque côté de chaque treillis de manière à limiter des efforts induits par le coulage du béton dans des directions perpendiculaires aux plans des treillis permet d'empêcher une déformation ou un déchirement d'un treillis pendant le coulage du béton.

L'utilisation d'une taille de maille supérieure à la taille maximale des granulats du béton permet aux granulats de pouvoir passer à travers le plan d'un treillis lors du coulage du béton, ce qui permet de garantir la continuité et l'homogénéité de l'échantillon de part et d'autre du plan de fissuration avant la fissuration. Ceci permet de produire un échantillon représentatif d'une dalle ou d'un corps en béton réel.

De préférence, chaque treillis est tendu sur le cadre de support respectif avant que celui-ci ne soit disposé sur le coffrage. Cependant, en variante, chaque cadre de support peut être disposé sur le coffrage avant que chaque treillis ne soit tendu sur le cadre de support respectif.

Selon un mode de réalisation, le procédé comprend en outre une étape supplémentaire, avant l'étape c), consistant à disposer au moins un outil vibrant au fond du coffrage et l'étape d) d'homogénéisation du béton consiste à vibrer le béton à l'aide de l'au moins un outil vibrant.

L'homogénéisation du béton à l'aide d'un outil vibrant disposé au fond du coffrage permet d'évacuer d'éventuelles bulles d'air introduites dans le béton lors du coulage et permet d'obtenir un échantillon homogène.

Selon un mode de réalisation, le procédé comprend en outre une étape supplémentaire, entre les étape d) et e), consistant à réaliser un surfaçage et une finition du béton au niveau de la face supérieure ouverte.

La réalisation d'un surfaçage et d'une étape de finition du béton au niveau de la face supérieure ouverte permet d'obtenir une surface plane représentative de conditions réelles d'utilisation, c'est-à-dire représentative d'une dalle ou d'un corps en béton mis en oeuvre sur une structure telle qu'un pont ou un bâtiment par exemple.

Selon un mode de réalisation, le procédé comprend en outre, entre les étapes b) et c), les étapes supplémentaires suivantes : réaliser au moins un ensemble d'ouvertures primaires dans l'au moins un treillis, les ouvertures d'un ensemble d'ouvertures primaires étant alignées suivant la direction sélectionnée, normale à l'au moins un treillis, lorsqu'il y a plusieurs treillis ; faire passer une armature horizontale par chaque ensemble d'ouvertures primaires ; fixer l'au moins une armature horizontale à l'aide d'au moins une armature transversale ; et disposer au moins un élément anti-adhérence sur l'au moins une armature horizontale à une intersection entre l'au moins une armature horizontale et un plan d'un treillis.

Les étapes supplémentaires décrites ci-dessus permettent de disposer des armatures dans l'échantillon tout en garantissant la propagation des fissures dans les plans de fissuration, et permettent donc d'obtenir un échantillon de béton armé fissuré avec une fissuration contrôlée.

Selon un mode de réalisation, le procédé comprend en outre, entre les étapes b) et c), les étapes supplémentaires suivantes : réaliser au moins une ouverture secondaire dans au moins un treillis ; positionner une fixation coulée en place à tester dans chaque ouverture secondaire ; fixer chaque fixation coulée en place à tester au cadre de support sur lequel est fixé le treillis dans lequel est formée l'ouverture secondaire respective ; et

le procédé comprenant en outre, à la suite de l'étape e), une étape supplémentaire consistant à libérer chaque fixation coulée en place à tester du cadre de support respectif.

Les étapes supplémentaires décrites ci-dessus permettent de disposer des fixations dites coulées en place dans l'échantillon de béton, ce qui permet par la suite de réaliser des essais mécaniques sur des fixations coulées en place disposées dans un plan de fissuration tel que cela est recommandé dans les guides d'essais.

Selon un mode de réalisation, les ouvertures secondaires sont réalisées, et les fixations coulées en place à tester sont positionnées, à proximité de la face supérieure ouverte.

Selon un mode de réalisation, le procédé comprend en outre, avant l'étape c), une étape supplémentaire consistant à mettre en place des fils de retenue dans chaque ouverture réalisée dans l'au moins un treillis de manière à empêcher une déformation de l'au moins un treillis à une ouverture lors de l'étape c) de coulage du béton.

L'utilisation de fils de retenue permet d'utiliser des ouvertures de la taille souhaitée dans le treillis, notamment des ouvertures suffisamment grandes autour des fixations coulées en place de manière à ce que le treillis ne se trouve pas à proximité directe de la fixation et que les essais mécaniques soient représentatifs, tout en garantissant que le treillis ne se déforme pas lors du coulage du béton.

Selon un mode de réalisation, le procédé comprend en outre, à la suite de l'étape g), les étapes supplémentaires suivantes : réaliser au moins un perçage dans au moins une face externe de l'échantillon de béton, chaque perçage étant réalisé perpendiculairement à la face externe correspondante et dans un plan de fissuration souhaité ; et implanter dans chaque perçage une fixation rapportée à tester.

Les étapes supplémentaires décrites ci-dessus permettent de disposer des fixations dites rapportées dans l'échantillon de béton, ce qui permet par la suite de réaliser des essais mécaniques sur des fixations rapportées disposées dans un plan de fissuration tel que cela est recommandé dans les guides d'essais.

Selon un mode de réalisation, les perçages sont réalisés, et les fixations rapportées à tester sont implantées, dans des faces externes de l'échantillon qui sont verticales au moins jusqu'à l'étape e).

Selon ce mode de réalisation, le coffrage est conçu et dimensionné de telle sorte que les faces de l'échantillon qui sont verticales lors du coulage sont représentatives et peuvent être utilisées pour réaliser des essais mécaniques sur des fixations. Ceci permet de rendre maximales les surfaces aptes à recevoir des fixations pour réaliser des essais représentatifs, et donc de rendre maximal le nombre de fixations qui peuvent être testées avec un même échantillon.

Selon un mode de réalisation, à l'état final, au moins un treillis s'étend dans l'échantillon sur tout le plan de fissuration souhaité correspondant sur toute la profondeur de l'échantillon, c'est-à-dire que des dimensions externes du treillis sont égales à des dimensions externes de l'échantillon lorsqu'ils sont vus en coupe transversale selon ledit plan de fissurations souhaité.

La disposition d'un treillis sur toute la section transversale de l'échantillon permet de guider la fissure de part en part de l'échantillon et permet de s'assurer de la propagation dans le seul plan de fissuration.

Selon un mode de réalisation, à l'état final, au moins un treillis s'étend à partir d'une surface externe de l'échantillon vers un coeur de celui-ci d'une distance égale à une profondeur d'ancrage d'une fixation disposée dans le plan dudit treillis.

La disposition d'un treillis sur la profondeur d'ancrage des fixations permet de garantir que la fissuration est bien comprise dans le plan de fissuration souhaité sur au moins toute la profondeur d'ancrage des fixations, comme cela est recommandé dans les guides d'essais.

Selon un mode de réalisation, l'initiation de la fissuration de l'étape g) comprend, pour au moins un plan de fissuration souhaité, l'utilisation de moyens d'expansion configurés pour appliquer une force entre des parties de l'échantillon situées de part et d'autre dudit plan de fissuration souhaité, de manière à mettre l'échantillon en traction.

L'utilisation de moyens d'expansion permet de facilement initier et propager une fissure dans un plan de fissuration, la fissure étant par la suite guidée par le treillis. Dans le cas où l'échantillon comprend des armatures horizontales, celles-ci sont de préférence mises en tractions pour promouvoir la propagation de la fissure.

Selon un mode de réalisation, le procédé comprend en outre une étape finale consistant à utiliser les moyens d'expansion pour commander la taille d'ouverture d'au moins une fissure.

L'utilisation des moyens d'expansion pour commander la taille d'ouverture de fissure permet notamment de réaliser des essais mécaniques à une ouverture de fissure contrôlée.

Selon un mode de réalisation, à l'état final, l'ouverture de fissure est comprise dans une plage allant de 0 à 1 mm, de préférence dans une plage allant de 0,1 à 0,8 mm. L'utilisation d'une ouverture de fissure comprise entre 0 et 1 mm permet de couvrir une plage représentative d'une fissuration réelle dans un corps en béton, et l'utilisation d'une ouverture de fissure comprise entre 0,1 et 0,8 mm permet de pouvoir réaliser des essais recommandés par les guides d'essais.

Selon un mode de réalisation, l'au moins un treillis est un treillis de fibres, de préférence de fibres de verre.

La présente invention a également pour objet un dispositif de production d'un échantillon de béton fissuré, comprenant un coffrage comprenant un fond horizontal et au moins trois banches latérales verticales pour former le coffrage à face supérieure ouverte, caractérisé par le fait qu'il comprend en outre au moins un cadre de support configuré pour être disposé vertical, transversalement au coffrage et pour y tendre un treillis, de manière à être configuré pour réaliser un procédé selon l'un des modes de réalisation décrits ci-dessus.

Le dispositif décrit ci-dessus permet de mettre en oeuvre le procédé selon la présente invention et permet la production d'un échantillon de béton fissuré par fissuration contrôlée, ledit échantillon étant configuré pour permettre la réalisation d'essais mécaniques sur des fixations coulées en place ou des fixations rapportées. Un dispositif selon un mode de réalisation de la présente invention et un procédé selon la présente invention vont maintenant être décrits à titre d'exemple non limitatif, avec référence aux dessins annexés.

Sur ces dessins :
[Fig. 1] est une vue en perspective d'un dispositif de production d'un échantillon de béton fissuré selon la présente invention, avec une vue éclatée d'un cadre de support selon la présente invention.
[Fig. 2] est une vue en coupe du dispositif selon la Figure 1, selon le plan du cadre de support central, et présente également une vue élargie du treillis.
[Fig. 3] est une vue de côté d'un échantillon de béton fissuré obtenu à l'aide d'un procédé et d'un dispositif selon la présente invention.

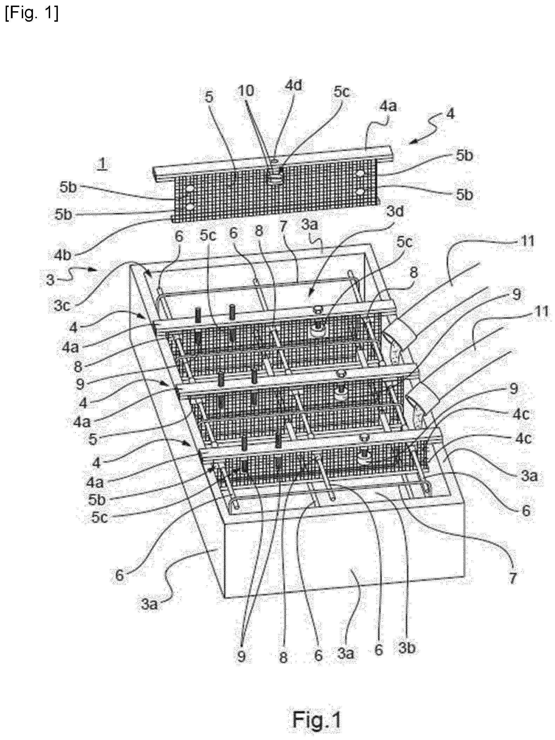

Si l'on se réfère tout d'abord à la Figure 1, on peut voir que l'on y a représenté un dispositif 1 de production d'un échantillon de béton fissuré 2. Le dispositif 1 comprend un coffrage 3 et des cadres de support 4 verticaux.

Le coffrage 3 comprend quatre banches latérales 3a disposées verticalement et un fond 3b horizontal, et le coffrage 3 est configuré de manière à laisser un face supérieure 3c ouverte.

L'utilisation de quatre banches latérales 3a permet de fabriquer un échantillon de béton fissuré 2 sous la forme d'un pavé droit, bien entendu un nombre différent de banches 3a peut être utilisé pour obtenir des échantillons 2 de différentes formes. Les banches latérales 3a et le fond 3b sont, de préférence, configurés de telle sorte que du béton n'adhère pas de manière définitive sur leur surface afin de permettre une séparation de l'échantillon de béton fissuré 2 et du coffrage 3 après un séchage du béton. Les banches latérales 3a sont de préférence réalisées en bois mais peuvent également être réalisées en un autre matériau comme par exemple de l'acier. Selon le mode de réalisation préféré, le fond 3b du coffrage 3 est formé par le sol, sur lequel peut éventuellement avoir été disposé un film ou un revêtement anti-adhérence. Selon une variante, le fond 3b peut également être réalisé à partir d'un élément en bois.

Sur la Figure 1 un cadre de support 4 supplémentaire a été représenté de manière éclatée afin de présenter plus clairement sa structure.

Chaque cadre de support 4 vertical comprend un élément transversal supérieur 4a auquel est fixé un treillis 5, et chaque cadre de support 4 vertical définit, en utilisation, un plan vertical, dit plan de fissuration, dans lequel s'étend le treillis 5 correspondant.

Selon le mode de réalisation préféré de la présente invention, le treillis 5 est un treillis de fibres de verre, mais pourrait en variante être constitué à base d'un autre matériau, comme par exemple du polyester.

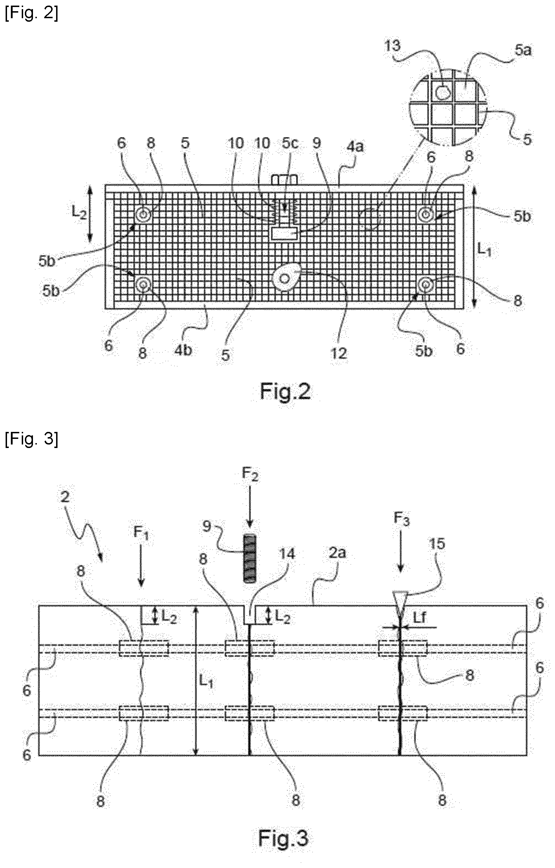

Selon le mode de réalisation représenté sur les Figures 1 et 2, chaque treillis 5 comprend des mailles 5a carrées. On comprendra cependant qu'en variante les mailles 5a peuvent avoir des formes différentes, par exemple polygonales.

En utilisation, chaque cadre de support 4 vertical est disposé transversalement à un espace intérieur 3d du coffrage 3 défini par les banches latérales 3a, le fond 3b et la face supérieure ouverte 3c, de telle sorte que le treillis 5 qui est fixé sur ledit cadre de support 4 s'étend transversalement audit espace intérieur 3d dans le plan vertical dit plan de fissuration respectif, c'est-à-dire un plan dans lequel on souhaite pouvoir guider une fissure dans l'échantillon 2.

De préférence, les dimensions des treillis 5 sont choisies de telle sorte que chaque treillis 5 s'étend, dans son plan de fissuration, d'une banche latérale 3a à une banche latérale 3a opposée.

Chaque cadre de support 4 est également disposé de telle sorte que tous les cadres de support 4 soient parallèles entre eux et perpendiculaires à une direction sélectionnée. Chaque cadre de support 4 est fixé au coffrage 3 afin d'être maintenu en position en utilisation.

Selon le mode de réalisation préféré, les cadres de support 4 sont réalisés en bois de telle sorte que les éléments transversaux 4a peuvent être cloués sur les banches latérales 3a. On comprendra cependant que les cadres de support 4 peuvent être réalisés dans d'autres matériaux, par exemple en métal, et que d'autres solutions de fixation, par exemple le soudage, le collage ou une fixation mécanique, peuvent être utilisées pour fixer les cadres de support 4 au coffrage 3.

Tel que cela est représenté en Figure 1, en utilisation, un treillis 5 peut s'étendre, à partir de la face supérieure ouverte 3c, sur la hauteur totale L1 du coffrage 3, ou sur une hauteur plus faible correspondant au moins à une distance L2, dite profondeur d'ancrage.

Lorsque le treillis 5 s'étend sur toute la hauteur L1 du coffrage 3, le cadre de support 4 comprend un élément transversal inférieur 4b, disposé au fond 3c du coffrage 3, auquel est fixée une extrémité inférieure du treillis 5 afin de tendre le treillis 5 entre l'élément transversal supérieur 4a et l'élément transversal inférieur 4b.

L'élément transversal supérieur 4a et l'élément transversal inférieur 4b peuvent chacun être conçus en une seule pièce sur laquelle le treillis 5 est fixé, par exemple par clouage, collage ou agrafage, ou en deux pièces qui permettent de pincer le treillis 5 pour le fixer. Lorsque l'élément transversal inférieur 4b est réalisé en une seule pièce, le treillis 5 peut aussi être fixé par pincement entre l'élément transversal inférieur 4b et le fond 3b du coffrage 3.

Lorsque le treillis 5 s'étend sur une hauteur L2 plus faible que la hauteur totale L1 du coffrage 3, le cadre de support 4 ne comprend pas d'élément transversal inférieur 4b et le treillis 5 est fixé, de manière à rester dans le plan vertical dit de fissuration, par des éléments de fixation latéraux 4c. On comprendra que, dans le cas où le treillis s'étend sur toute la hauteur L1, des éléments de fixation latéraux 4c peuvent également être utilisés en complément de l'élément transversal inférieur 4b.

Selon le mode de réalisation représenté sur la Figure 1, les éléments de fixation latéraux 4c se présentent sous la forme d'agrafes reliées aux banches latérales 3a. Cependant, on comprendra que les éléments de fixation latéraux 4c peuvent, en variante, prendre d'autres formes comme par exemple des points de colle, on pourrait également réaliser des rainures verticales dans les banches latérales 3a dans lesquelles seraient reçues puis bloquées des baguettes de tailles correspondantes afin de pincer le treillis 5 et de maintenir le treillis 5 en position. Selon une variante du mode de réalisation représenté sur les Figures, les cadres de support 4 pourraient également comprendre des éléments de support latéraux verticaux à la périphérie des treillis 5, afin de faciliter la disposition des treillis 5 sur les cadres de support 4, de faciliter la fixation des treillis 5 aux cadres de support 4, et de permettre une éventuelle disposition des treillis 5 sur les cadres de support 4 avant une disposition des cadres de support 4 sur le coffrage 3.

Selon le mode de réalisation préféré représenté sur les Figures 1 et 2, des armatures horizontales 6 s'étendent d'une extrémité à l'autre du coffrage 3 suivant la direction sélectionnée, et donc perpendiculairement aux plans des treillis 5.

Les armatures horizontales 6 sont maintenues en position grâce à des armatures transversales 7, comme cela est déjà connu pour la mise en oeuvre d'un béton armé. Afin de permettre le passage des armatures horizontales 6 à travers les treillis 5, des ouvertures primaires 5b alignées suivant la direction sélectionnée sont réalisées dans les treillis 5. Selon le mode de réalisation représenté sur les Figures 1 et 2, quatre armatures horizontales 6 sont prévues. On comprendra cependant qu'un autre nombre d'armatures horizontales 6 peut être sélectionné pour l'échantillon 2. On comprendra également que, selon le mode de réalisation représenté sur la Figure 1, la direction sélectionnée correspond à un direction de longueur du coffrage 3, mais que la direction sélectionnée pourrait également, en variante, correspondre à une autre direction du coffrage 3, notamment une direction de largeur.

Comme cela est représenté sur les Figures 1 à 3, des éléments anti-adhérence 8 sont disposés sur chaque armature horizontale 6 au niveau des intersections entre ladite armature horizontale 6 et le plan vertical de chaque treillis 5 afin d'empêcher une adhérence entre du béton et ladite armature horizontale 6 dans ces zones. De tels éléments anti-adhérence 8 sont déjà connus et ne seront pas décrits en détail. Le dispositif 1 permet la mise en place de fixations 9 coulées en place dans l'échantillon 2. Comme on peut le voir sur les Figures 1 et 2, des ouvertures secondaires 5c sont réalisées dans certains treillis 5. Une ouverture secondaire 5c est réalisée dans un treillis 5 de manière à permettre la disposition d'une fixation 9 coulée en place. Comme on peut le voir sur la Figure 2, les dimensions d'une ouverture secondaire 5c sont supérieures aux dimensions de la fixation 9 coulée en place correspondante, de manière à pouvoir disposer la fixation 9 coulée en place dans le plan du treillis 5 correspondant et de manière à ce que des essais mécaniques réalisés sur la fixation 9 coulée en place, une fois l'échantillon 2 produit, ne soient pas faussés par une proximité du treillis 5 avec la fixation 9 coulée en place.

Chaque fixation 9 coulée en place est maintenue en position à l'aide du cadre de support 4 correspondant de manière à ce qu'à l'état final elle s'étende de la longueur L2, dite profondeur d'ancrage, dans l'échantillon 2 vers le coeur de l'échantillon 2. De manière préférée, un trou traversant 4d est réalisé dans l'élément transversal supérieur 4a du cadre de support 4 respectif et la fixation 9 coulée en place est passée à travers le trou traversant 4d et est maintenue en position par un élément de blocage. L'élément de blocage peut par exemple prendre la forme d'un anneau de serrage.

Des fils de retenue 10 sont disposés autour de chaque ouverture secondaire 5c et passent à travers des mailles 5a du treillis 5, de manière à ce que le treillis 5 ne se déforme pas lors d'un coulage de béton dans le coffrage 3. On comprendra que de la même manière et pour les mêmes raisons, des fils de retenue 10 peuvent être mis en place autour de chaque ouverture primaire 5b.

Deux tuyaux d'alimentation en béton 11 sont prévus pour couler du béton dans le coffrage 3. De préférence, le coulage du béton se fait de manière équilibrée de chaque côté de chaque treillis 5 de manière à limiter des efforts induits par le coulage du béton dans des directions perpendiculaires aux plans des treillis 5, afin de limiter une déformation des treillis 5 pendant le coulage du béton. On comprendra qu'un seul tuyau d'alimentation en béton 11 peut être utilisé ou encore que le béton puisse être amené d'une autre manière, comme par exemple par pelletage.

Comme représenté en Figure 2, un outil vibrant 12 est également prévu pour homogénéiser le béton après coulage et pour évacuer d'éventuelles bulles d'air introduites dans le béton lors du coulage. En variante d'autres solutions pourraient également être utilisées, comme par exemple mettre en place le dispositif 1 sur une table vibrante.

Comme cela est représenté schématiquement sur la vue élargie de la Figure 2, les mailles 5a des treillis 5 sont configurées en fonction du béton utilisé, de manière à ce qu'une dimension des granulats 13 du béton soit inférieure à une dimension des mailles 5a. Ceci permet, lors du coulage du béton, aux granulats 13 du béton de pouvoir traverser le plan d'un treillis de manière à garantir la continuité et l'homogénéité de l'échantillon 2 de part et d'autre du plan dit de fissuration, avant une fissuration.

La Figure 3 représente une vue de côté d'un échantillon de béton fissuré 2 fabriqué à l'aide d'un dispositif 1 selon la présente invention, les armatures horizontales 6 et les éléments anti-adhérence 8 sont représentés par transparence en pointillés. La première fissure F1 représente une fissure initiée puis guidée par un treillis 5 s'étendant sur la longueur L2 dans un premier plan de fissuration vertical. La deuxième fissure F2 représente une fissure initiée puis guidée par un treillis 5 s'étendant sur la longueur L1 dans un deuxième plan de fissuration vertical. On peut voir qu'un perçage 14 a été réalisé sur une longueur L2, perpendiculairement à une surface externe 2a et de manière centrée sur le deuxième plan de fissuration vertical. Ce perçage 14 est destiné à recevoir une fixation 9 rapportée qui sera scellée dans l'échantillon 2 de manière mécanique ou chimique. Dans le cas d'un scellement mécanique, la liaison entre la fixation 9 rapportée et le béton est assurée par un frottement ou une expansion d'au moins un élément mécanique, ledit au moins un élément mécanique étant généralement en acier. Dans le cas d'un scellement chimique, la liaison entre la fixation 9 rapportée et le béton est assurée par adhérence, de préférence à l'aide d'un mortier polymère ou cimentaire.

De préférence, les perçages 14 sont réalisés dans des surfaces externes 2a de l'échantillon 2 qui sont verticales pendant le coulage du béton, de telle sorte que des fixations 9 rapportées peuvent être mises en place successivement sur deux côtés opposés de l'échantillons 2 et que les essais mécaniques réalisés sur lesdites fixations 9 rapportées peuvent être considérés comme ayant été réalisés dans des conditions identiques. Cela permet de maximiser le nombre de fixations 9 testées pour un même échantillon de béton 2.

La troisième fissure F3 représente une fissure qui vient d'être initiée par des moyens d'expansion 15, qui peuvent par exemple se présenter sous la forme d'un coin, c'est-à-dire un élément d'expansion conique qui écarte les bords de la fissure lorsqu'un impact lui est appliqué.

Lesdits moyens d'expansion 15 peuvent également être utilisés pour contrôler une ouverture de fissure Lf pendant un essai mécanique réalisé sur une fixation 9, ladite fixation 9 pouvant être une fixation 9 coulée en place ou une fixation 9 rapportée.

De préférence, le dispositif permet de contrôler l'ouverture de fissure Lf entre 0 et 1 mm, et de préférence encore entre 0,3 et 0,8 mm.

Les fixations 9 représentées sur les Figures 1 et 2 sont des fixations 9 comprenant un ancrage unique, on comprendra cependant que tous les types de fixations 9 peuvent être utilisés, comme par exemple des rails de fixation qui comprennent plusieurs points d'ancrage.

On va maintenant décrire un procédé de fabrication d'un échantillon de béton fissuré 2 selon la présente invention. Les différents éléments nécessaires à la mise en oeuvre du procédé ont été décrits en détail lors de la description du dispositif selon la présente invention et leurs descriptions ne seront donc pas reprises en détail ci-dessous.

Le procédé selon la présente invention comprend tout d'abord mettre en place un coffrage 3 selon des dimensions souhaitées pour l'échantillon 2. Le coffrage 3 comprend un fond 3b horizontal et au moins quatre banches 3a latérales verticales formant avec le fond 3b, le coffrage 3 avec une face supérieure ouverte 3c.

Le procédé comprend ensuite disposer au moins un cadre de support 4 vertical transversalement au coffrage 3, l'au moins un cadre de support 4 vertical s'étendant sur tout ou partie de la profondeur du coffrage 3. Selon le procédé, un treillis 5 est tendu sur chaque cadre de support 4 dans le plan de fissuration vertical correspondant, chaque cadre de support 4 vertical définit un plan de fissuration vertical, et chaque plan de fissuration est perpendiculaire à une direction sélectionnée.

De préférence, l'au moins un treillis est un treillis de fibres, de préférence encore de fibres de verre.

De manière préféré le procédé comprend disposer au moins un outil vibrant 12 au fond du coffrage 3.

De manière facultative, le procédé comprend mettre en place des armatures horizontales 6. Le procédé comprend alors réaliser au moins un ensemble d'ouvertures primaires 5b dans l'au moins un treillis 5, les ouvertures 5b d'un ensemble d'ouvertures primaires 5b étant alignées suivant la direction sélectionnée, normale à l'au moins un treillis 5, lorsqu'il y a plusieurs treillis 5, puis faire passer une armature horizontale 6 par chaque ensemble d'ouvertures primaires 5b, puis fixer l'au moins une armature horizontale 6 à l'aide d'au moins une armature transversale 7, et enfin disposer au moins un élément anti-adhérence 8 sur l'au moins une armature horizontale 6 à une intersection entre l'au moins une armature horizontale 6 et un plan d'un treillis 5.

On comprendra que les étapes ci-dessus sont effectuées uniquement dans le cas où l'on souhaite réaliser un échantillon 2 en béton armé.

De manière facultative encore, le procédé comprend mettre en place des fixations 9 dites coulées en place. Le procédé comprend alors réaliser au moins une ouverture secondaire 5c dans au moins un treillis 5, puis positionner une fixation 9 coulée en place à tester dans chaque ouverture secondaire 5c, et enfin fixer chaque fixation 9 coulée en place à tester au cadre de support 4 sur lequel est fixé le treillis 5 dans lequel est formée l'ouverture secondaire 5c respective.

De manière préférée, les ouvertures secondaires 5c sont réalisées, et les fixations 9 coulées en place à tester sont positionnées, à proximité de la face supérieure ouverte 3c.

On comprendra que les étapes ci-dessus sont effectuées uniquement dans le cas où l'on souhaite mettre en place des fixations 9 coulées en place dans l'échantillon 2 de manière à pouvoir par la suite réaliser des essais mécaniques sur lesdites fixations 9 coulées en place.

De manière préférée, le procédé comprend une étape consistant à mettre en place des fils de retenue 10 dans chaque ouverture 5b, 5c réalisée dans l'au moins un treillis 5 de manière à empêcher une déformation de l'au moins un treillis 5 à une ouverture 5b, 5c lors du futur coulage de béton.

Après quoi le procédé comprend couler du béton dans le coffrage par la face supérieure ouverte pour remplir le coffrage.

De manière préférée, selon le procédé, le coulage du béton se fait par la face supérieure ouverte 3c et de manière équilibrée de chaque côté de chaque treillis 5 de manière à limiter des efforts induits par le coulage du béton dans des directions perpendiculaires aux plans des treillis 5.

Après cela, le procédé comprend homogénéiser le béton dans le coffrage.

De manière préférée, l'homogénéisation du béton consiste à vibrer le béton à l'aide d'au moins un outil vibrant 12.

De manière préféré encore, le procédé comprend de plus réaliser un surfaçage et une finition du béton au niveau de la face supérieure ouverte.

Le procédé comprend ensuite attendre pendant une période prédéfinie pour le séchage du béton.

On comprendra qu'un fois le séchage du béton obtenu, le coffrage 3 et l'échantillon 2 peuvent être librement manipulés, déplacés ou tournés selon les besoins.

Dans le cas, facultatif, où l'on a mis en place des fixations 9 rapportées dans l'échantillon 2, on comprendra que le procédé comprend alors une étape consistant à libérer chaque fixation 9 coulée en place à tester du cadre de support 4 respectif. Puis le procédé comprend retirer le coffrage et l'au moins un cadre de support.

On comprendra qu'un fois le coffrage 3 retiré, l'échantillon 2 peut être librement manipulé, déplacé ou tourné selon les besoins.

Par la suite le procédé comprend initier une fissuration contrôlée dans chaque plan de fissuration souhaité de l'échantillon.

De manière préférée, l'initiation de la fissuration comprend, pour au moins un plan de fissuration souhaité, l'utilisation de moyens d'expansion 15 configurés pour appliquer une force entre des parties de l'échantillon 2 situées de part et d'autre dudit plan de fissuration souhaité, de manière à mettre l'échantillon 2 en traction.

On comprendra que des perçages peuvent éventuellement être réalisés dans l'échantillon 2 pour permettre l'utilisation desdits moyens d'expansion.

Dans le cas où l'échantillon comprend des armatures horizontales, celles-ci sont de préférence mises en tractions pour promouvoir la propagation de la fissure.

De manière facultative, le procédé peut en outre comprendre mettre en place des fixations 9 dites rapportées. Le procédé comprend alors réaliser au moins un perçage 14 dans au moins une face externe 2a de l'échantillon 2 de béton, chaque perçage 14 étant réalisé perpendiculairement à la face externe 2a correspondante et dans un plan de fissuration souhaité, puis implanter dans chaque perçage 14 une fixation 9 rapportée à tester.

De manière préférée selon le procédé, les perçages 14 sont réalisés, et les fixations 9 rapportées à tester sont implantées, dans des faces externes 2a de l'échantillon 2 qui sont verticales au moins jusqu'à l'étape de séchage du béton.

On comprendra que les étapes ci-dessus sont effectuées uniquement dans le cas où l'on souhaite mettre en place des fixations 9 rapportées dans l'échantillon 2 de manière à pouvoir par la suite réaliser des essais mécaniques sur lesdites fixations 9 rapportées.

On comprendra également que, selon les besoins, l'échantillon 2 peut être retourné pour faciliter la réalisation des perçages 14 et l'implantation des fixations 9 rapportées ou bien encore pour faciliter la réalisation des essais mécaniques sur les fixations 9.

De manière préférée, le procédé comprend ensuite utiliser les moyens d'expansion pour commander la taille d'ouverture Lf d'au moins une fissure.

On comprendra que cette étape permet de contrôler les ouvertures de fissure Lf de l'échantillon 2 lors des essais mécaniques réalisés sur les fixations 9, qu'il s'agisse de fixations 9 coulées en place ou de fixations 9 rapportées.

De manière préférée, le procédé est configuré pour qu'à l'état final, l'ouverture de fissure Lf soit comprise dans une plage allant de 0 à 1 mm, et de préférence encore dans une plage allant de 0,3 à 0,8 mm.

De manière préférée, selon le procédé, les dimensions des treillis 5 sont choisies de telle sorte que chaque treillis 5 s'étend dans son plan de fissuration d'une banche latérale 3a à une banche latérale 3a opposée.

De manière préférée encore, selon le procédé, le béton utilisé pour fabriquer l'échantillon 2 comprend des granulats 13 et une dimension de maille 5a de l'au moins un treillis 5 est supérieure à une dimension maximale des granulats 13.

De manière préférée encore, le procédé est prévu de telle sorte qu'à l'état final, au moins un treillis 5 s'étend dans l'échantillon 2 sur tout le plan de fissuration souhaité correspondant sur toute la profondeur L1 de l'échantillon 2, c'est-à-dire que des dimensions externes du treillis 5 sont égales à des dimensions externes de l'échantillon 2 lorsqu'ils sont vus en coupe transversale selon ledit plan de fissurations souhaité.

En variante, le procédé peut être prévu de telle sorte qu'à l'état final, au moins un treillis 5 s'étend à partir d'une surface externe 2a de l'échantillon vers un coeur de celui-ci d'une distance L2 égale à une profondeur d'ancrage d'une fixation 9 disposée dans le plan dudit treillis 5.

On comprendra enfin que le procédé selon la présente invention permet une utilisation de différents types de fixations pour les fixations 9 coulées en place et pour les fixations 9 rapportées, comme par exemple des fixations à ancrage unique ou des fixations avec plusieurs points d'ancrage, par exemple des plaques de connexion ou des rails d'ancrage.

Le procédé selon l'invention permet donc d'obtenir un échantillon de béton fissuré 2 par fissuration contrôlée. Ledit échantillon 2 peut être en béton ou en béton armé et peut comprendre des fixations 9 coulées en place ou des fixation 9 rapportées. Lesdites fixations 9 sont disposées, de manière précise et garantie, verticalement dans des plans de fissuration afin de permettre de réaliser des essais mécaniques sur lesdites fixations 9.

## Revendications

1. Procédé de production d'un échantillon de béton fissuré (2) par fissuration contrôlée, comprenant les étapes suivantes :
a) mettre en place un coffrage (3) selon des dimensions souhaitées pour l'échantillon (2), le coffrage (3) comprenant un fond (3b) horizontal et au moins trois banches latérales (3a) verticales formant avec le fond (3b) le coffrage (3) avec une face supérieure ouverte (3c) ;
b) disposer au moins un cadre de support (4) vertical transversalement au coffrage (3), l'au moins un cadre de support (4) vertical s'étendant sur tout ou partie de la profondeur du coffrage (3), un treillis (5) étant tendu sur chaque cadre de support (4) dans un plan de fissuration vertical correspondant, chaque cadre de support (4) vertical définissant un plan de fissuration vertical, chaque plan de fissuration étant perpendiculaire à une direction sélectionnée ;
c) couler du béton dans le coffrage (3) par la face supérieure ouverte (3c) pour remplir le coffrage, le coulage du béton se faisant de manière équilibrée de chaque côté de chaque treillis (5) de manière à limiter des efforts induits par le coulage du béton dans des directions perpendiculaires aux plans des treillis (5), le béton comprenant des granulats (13) et une dimension de maille (5a) de l'au moins un treillis (5) étant supérieure à une dimension maximale des granulats (13) ;
d) homogénéiser le béton dans le coffrage (3) ;
e) attendre pendant une période prédéfinie pour le séchage du béton ;
f) retirer le coffrage (3) et l'au moins un cadre de support (4) ; et
g) initier une fissuration contrôlée dans chaque plan de fissuration souhaité de l'échantillon.

2. Procédé selon la revendication 1, **caractérisé par le fait qu'**il comprend en outre une étape supplémentaire, avant l'étape c), consistant à disposer au moins un outil vibrant (12) au fond du coffrage (3) et l'étape d) d'homogénéisation du béton consiste à vibrer le béton à l'aide de l'au moins un outil vibrant (12).

3. Procédé selon la revendication 1 ou la revendication 2, **caractérisé par le fait que** le procédé comprend en outre une étape supplémentaire, entre les étape d) et e), consistant à réaliser un surfaçage et une finition du béton au niveau de la face supérieure ouverte (3c).

4. Procédé selon l'une quelconque des revendications 1 à 3, **caractérisé par le fait qu'**il comprend en outre, entre les étapes b) et c), les étapes supplémentaires suivantes :
- réaliser au moins un ensemble d'ouvertures primaires (5b) dans l'au moins un treillis (5), les ouvertures (5b) d'un ensemble d'ouvertures primaires (5b) étant alignées suivant la direction sélectionnée, normale à l'au moins un treillis (5), lorsqu'il y a plusieurs treillis ;
- faire passer une armature horizontale (6) par chaque ensemble d'ouvertures primaires (5b) ;
- fixer l'au moins une armature horizontale (6) à l'aide d'au moins une armature transversale (7) ; et
- disposer au moins un élément anti-adhérence (8) sur l'au moins une armature horizontale (6) à une intersection entre l'au moins une armature horizontale (6) et un plan d'un treillis (5).

5. Procédé selon l'une quelconque des revendication 1 à 4, **caractérisé par le fait qu'**il comprend en outre, entre les étapes b) et c), les étapes supplémentaires suivantes :
- réaliser au moins une ouverture secondaire (5c) dans au moins un treillis (5) ;
- positionner une fixation (9) coulée en place à tester dans chaque ouverture secondaire (5c) ;
- fixer chaque fixation (9) coulée en place à tester au cadre de support (4) sur lequel est fixé le treillis (5) dans lequel est formée l'ouverture secondaire (5c) respective ; et le procédé comprenant en outre, à la suite de l'étape e), une étape supplémentaire consistant à libérer chaque fixation (9) coulée en place à tester du cadre de support (4) respectif.

6. Procédé selon la revendication 5, **caractérisé par le fait que** les ouvertures secondaires (5c) sont réalisées, et les fixations (9) coulées en place à tester sont positionnées, à proximité de la face supérieure ouverte (3c).

7. Procédé selon l'une quelconque des revendications 4 à 6, **caractérisé par le fait qu'**il comprend en outre, avant l'étape c), une étape supplémentaire consistant à mettre en place des fils de retenue (10) dans chaque ouverture (5b, 5c) réalisée dans l'au moins un treillis (5) de manière à empêcher une déformation de l'au moins un treillis (5) à une ouverture (5b, 5c) lors de l'étape c) de coulage du béton.

8. Procédé selon l'une quelconque des revendications 1 à 7, **caractérisé par le fait qu'**il comprend en outre, à la suite de l'étape g), les étapes supplémentaires suivantes :
- réaliser au moins un perçage (14) dans au moins une face externe (2a) de l'échantillon de béton (2), chaque perçage (14) étant réalisé perpendiculairement à la face externe (2a) correspondante et dans un plan de fissuration souhaité ; et
- implanter dans chaque perçage (14) une fixation (9) rapportée à tester.

9. Procédé selon la revendication 8, **caractérisé par le fait que** les perçages (14) sont réalisés, et les fixations (9) rapportées à tester sont implantées, dans des faces externes (2a) de l'échantillon (2) qui sont verticales au moins jusqu'à l'étape e).

10. Procédé selon l'une quelconque des revendications 1 à 9, **caractérisé par le fait qu'**à l'état final, au moins un treillis (5) s'étend dans l'échantillon (2) sur tout le plan de fissuration souhaité correspondant sur toute la profondeur (L1) de l'échantillon (2), c'est-à-dire que des dimensions externes du treillis (5) sont égales à des dimensions externes de l'échantillon (2) lorsqu'ils sont vus en coupe transversale selon ledit plan de fissurations souhaité.

11. Procédé selon la revendication 5 ou 8, ou l'une quelconque des revendications 6, 7 ou 9, lorsqu'elle dépend de la revendication 5 ou 8, **caractérisé par le fait qu'**à l'état final, au moins un treillis (5) s'étend à partir d'une surface externe (2a) de l'échantillon (2) vers un coeur de celui-ci d'une distance égale à une profondeur d'ancrage (L2) d'une fixation (9) disposée dans le plan dudit treillis (5).

12. Procédé selon l'une quelconques des revendications 1 à 11, **caractérisé par le fait que** l'initiation de la fissuration de l'étape g) comprend, pour au moins un plan de fissuration souhaité, l'utilisation de moyens d'expansion (15) configurés pour appliquer une force entre des parties de l'échantillon (2) situées de part et d'autre dudit plan de fissuration souhaité, de manière à mettre l'échantillon (2) en traction.

13. Procédé selon la revendication 12, **caractérisé par le fait qu'**il comprend en outre une étape finale consistant à utiliser les moyens d'expansion (15) pour commander la taille d'ouverture (Lf) d'au moins une fissure.

14. Procédé selon l'une quelconque des revendications 1 à 13, **caractérisé par le fait qu'**à l'état final, l'ouverture de fissure (Lf) est comprise dans une plage allant de 0 à 1 mm, de préférence dans une plage allant de 0,1 à 0,8 mm.

15. Procédé selon l'une quelconque des revendications 1 à 14, **caractérisé par le fait que** l'au moins un treillis (5) est un treillis de fibres, de préférence de fibres de verre.

16. Dispositif (1) de production d'un échantillon de béton fissuré (2), comprenant un coffrage (3) comprenant un fond (3b) horizontal et au moins trois banches latérales (3a) verticales pour former le coffrage (3) à face supérieure ouverte (3c), **caractérisé par le fait qu'**il comprend en outre au moins un cadre de support (4) configuré pour être disposé vertical, transversalement au coffrage (3) et pour y tendre un treillis (5), de manière à être configuré pour réaliser un procédé selon l'une quelconque des revendications 1 à 15.

## Patentansprüche

1. - Verfahren zur Herstellung einer gerissenen Betonprobe (2) durch kontrollierte Rissbildung, umfassend die folgenden Schritte:
a) Anbringen einer Schalung (3) gemäß den gewünschten Abmessungen für die Probe (2), die Schalung (3) umfassend einen horizontalen Boden (3b) und mindestens drei vertikale seitliche Schalwände (3a), die mit dem Boden (3b) die Schalung (3) mit einer offenen Oberseite (3c) bilden;
b) Anordnen mindestens eines vertikalen Stützrahmens (4) quer zu der Schalung (3), wobei sich der mindestens eine vertikale Stützrahmen (4) über die gesamte oder einen Teil der Tiefe der Schalung (3) erstreckt, wobei ein Gitter (5) in einer entsprechenden vertikalen Rissbildungsebene über jeden Stützrahmen (4) gespannt ist, wobei jeder vertikale Stützrahmen (4) eine vertikale Rissbildungsebene definiert, wobei jede Rissbildungsebene senkrecht zu einer ausgewählten Richtung ist;
c) Gießen des Betons in die Schalung (3) an der offenen Oberseite (3c), um die Schalung zu füllen, wobei das Gießen des Betons ausgeglichen auf jeder Seite jedes Gitters (5) erfolgt, um Kräfte zu begrenzen, die durch das Gießen des Betons in Richtungen senkrecht zu den Ebenen der Gitter (5) induziert werden, der Beton umfassend Zuschlagstoffe (13) und wobei eine Maschengröße (5a) des mindestens einen Gitters (5) größer ist als eine maximale Abmessung der Zuschlagstoffe (13) ;
d) Homogenisieren des Betons in der Schalung (3);
e) Warten während eines vordefinierten Zeitraums, bis der Beton getrocknet ist;
f) Entfernen der Schalung (3) und des mindestens einen Stützrahmens (4); und
g) Einleiten einer kontrollierten Rissbildung in jeder gewünschten Rissbildungsebene der Probe.

2. - Verfahren nach Anspruch 1, **dadurch gekennzeichnet, dass** es ferner einen ergänzenden Schritt vor Schritt c) umfasst, der darin besteht, mindestens ein vibrierendes Werkzeug (12) an dem Boden der Schalung (3) anzuordnen, und Schritt d) einer Homogenisierung des Betons darin besteht, den Beton mittels des mindestens einen vibrierenden Werkzeugs (12) zu vibrieren.

3. - Verfahren nach Anspruch 1 oder 2, **dadurch gekennzeichnet, dass** das Verfahren ferner einen ergänzenden Schritt zwischen Schritt d) und e) umfasst, der darin besteht, eine Oberflächenbearbeitung und eine Endbearbeitung des Betons auf Ebene der offenen Oberseite (3c) vorzunehmen.

4. - Verfahren nach einem der Ansprüche 1 bis 3, **dadurch gekennzeichnet, dass** es zwischen Schritt b) und c) ferner die folgenden ergänzenden Schritte umfasst:
- Anfertigen mindestens einer Anordnung von Hauptöffnungen (5b) in dem mindestens einen Gitter (5), wobei die Öffnungen (5b) einer Anordnung von Hauptöffnungen (5b) entlang der ausgewählten Richtung senkrecht zu dem mindestens einen Gitter (5) ausgerichtet sind, wenn es mehrere Gitter gibt;
- Durchführen einer horizontalen Bewehrung (6) durch jede Anordnung von Hauptöffnungen (5b);
- Befestigen der mindestens einen horizontalen Bewehrung (6) mittels mindestens einer Querbewehrung (7); und
- Anordnen mindestens eines Haftschutzelements (8) auf der mindestens einen horizontalen Bewehrung (6) an einem Schnittpunkt zwischen der mindestens einen horizontalen Bewehrung (6) und einer Ebene eines Gitters (5).

5. - Verfahren nach einem der Ansprüche 1 bis 4, **dadurch gekennzeichnet, dass** es zwischen Schritt b) und c) ferner die folgenden ergänzenden Schritte umfasst:
- Anfertigen mindestens einer Sekundäröffnung (5c) in mindestens einem Gitter (5);
- Positionieren einer zu testenden, vor Ort gegossenen Befestigung (9) in jeder Sekundäröffnung (5c);
- Befestigen jeder zu testenden, vor Ort gegossenen Befestigung (9) an dem Stützrahmen (4), an dem das Gitter (5) befestigt ist, in dem die jeweilige Sekundäröffnung (5c) gebildet ist; und wobei das Verfahren ferner nach Schritt e) einen ergänzenden Schritt eines Freigebens von jeder zu testenden, vor Ort gegossenen Befestigung (9) von dem jeweiligen Stützrahmen (4) umfasst.

6. - Verfahren nach Anspruch 5, **dadurch gekennzeichnet, dass** die Sekundäröffnungen (5c) in der Nähe der offenen Oberseite (3c) ausgeführt werden und die zu testenden, vor Ort gegossenen Befestigungen (9) dort positioniert werden.

7. - Verfahren nach einem der Ansprüche 4 bis 6, **dadurch gekennzeichnet, dass** es ferner vor Schritt c) einen ergänzenden Schritt umfasst, der darin besteht, Haltedrähte (10) in jeder Öffnung (5b, 5c) anzubringen, die in dem mindestens einen Gitter (5) angefertigt ist, umfasst, um eine Verformung des mindestens einen Gitters (5) an einer Öffnung (5b, 5c) während Schritt c) eines Gießens des Betons zu verhindern.

8. - Verfahren nach einem der Ansprüche 1 bis 7, **dadurch gekennzeichnet, dass** es ferner nach Schritt g) die folgenden ergänzenden Schritte umfasst:
- Anfertigen mindestens einer Bohrung (14) in mindestens einer Außenseite (2a) der Betonprobe (2), wobei jede Bohrung (14) senkrecht zu der entsprechenden Außenseite (2a) und in einer gewünschten Rissbildungsebene angefertigt wird; und
- Einführen einer zu testenden, eingebrachten Befestigung (9) in jede Bohrung (14).

9. - Verfahren nach Anspruch 8, **dadurch gekennzeichnet, dass** die Bohrungen (14) in den Außenseiten (2a) der Probe (2), die zumindest bis zum Schritt e) vertikal sind, angefertigt und die zu testenden, eingebrachten Befestigungen (9) darin eingeführt werden.

10. - Verfahren nach einem der Ansprüche 1 bis 9, **dadurch gekennzeichnet, dass** sich im Endzustand mindestens ein Gitter (5) in der Probe (2) über die gesamte entsprechende gewünschte Rissbildungsebene, über die gesamten Tiefe (L1) der Probe (2) erstreckt, soll heißen, die Außenabmessungen des Gitters (5) sind gleich wie die Außenabmessungen der Probe (2), wenn sie im Querschnitt entlang der gewünschten Rissbildungsebene betrachtet werden.

11. - Verfahren nach Anspruch 5 oder 8 oder einem der Ansprüche 6, 7 oder 9, wenn abhängig von Anspruch 5 oder 8, **dadurch gekennzeichnet, dass** sich im Endzustand mindestens ein Gitter (5) von einer Außenfläche (2a) der Probe (2) zu einem Kern derselben um eine Strecke erstreckt, die gleich ist wie eine Verankerungstiefe (L2) einer Befestigung (9), die in der Ebene des Gitters (5) angeordnet ist.

12. - Verfahren nach einem der Ansprüche 1 bis 11, **dadurch gekennzeichnet, dass** das Einleiten der Rissbildung in Schritt g) für mindestens eine gewünschte Rissbildungsebene die Verwendung von Expansionseinrichtungen (15) umfasst, die konfiguriert sind, um eine Kraft zwischen Teilen der Probe (2) anzuwenden, die sich auf beiden Seiten der gewünschten Rissbildungsebene befinden, um die Probe (2) Zug auszusetzen.

13. - Verfahren nach Anspruch 12, **dadurch gekennzeichnet, dass** es ferner einen abschließenden Schritt umfasst, der darin besteht, die Expansionseinrichtungen (15) zu verwenden, um die Öffnungsgröße (Lf) von mindestens einem Riss zu steuern.

14. - Verfahren nach einem der Ansprüche 1 bis 13, **dadurch gekennzeichnet, dass** die Rissöffnung (Lf) im Endzustand in einem Bereich von 0 bis 1 mm, vorzugsweise in einem Bereich von 0,1 bis 0,8 mm, liegt.

15. - Verfahren nach einem der Ansprüche 1 bis 14, **dadurch gekennzeichnet, dass** das mindestens eine Gitter (5) ein Gitter aus Fasern, vorzugsweise aus Glasfasern, ist.

16. - Vorrichtung (1) zur Herstellung einer Probe aus gerissenem Beton (2), umfassend eine Schalung (3), umfassend einen horizontalen Boden (3b) und mindestens drei vertikale seitliche Schalwände (3a), um die Schalung (3) mit offener Oberseite (3c) zu bilden, **dadurch gekennzeichnet, dass** sie ferner mindestens einen Stützrahmen (4) umfasst, der konfiguriert ist, um vertikal quer zu der Schalung (3) angeordnet zu sein und ein Gitter (5) darin zu spannen, sodass sie konfiguriert ist, um ein Verfahren nach einem der Ansprüche 1 bis 15 durchzuführen.

## Claims

1. - A method of producing a cracked concrete sample (2) by controlled cracking, comprising the following steps:
a) placing a formwork (3) with the desired dimensions for the sample (2), the formwork (3) comprising a horizontal base (3b) and at least three vertical side panels (3a) forming, with the base (3b), the formwork (3) with an open upper face (3c) ;
b) arranging at least one vertical support frame (4) transverse to the formwork (3), the at least one vertical support frame (4) extending over all or part of the depth of the formwork (3), a mesh (5) being stretched over each support frame (4) in a corresponding vertical cracking plane, each vertical support frame (4) defining a vertical cracking plane, each cracking plane being perpendicular to a selected direction;
c) pouring concrete into the formwork (3) through the open upper face (3c) to fill the formwork, the concrete pouring taking place in a balanced manner on each side of each mesh (5) so as to limit forces induced by the concrete pouring in directions perpendicular to the planes of the meshes (5), the concrete comprising aggregates (13) and a mesh size (5a) of the at least one mesh (5) being greater than a maximum size of the aggregates (13);
d) homogenizing the concrete in the formwork (3);
e) waiting for a predefined period for the concrete to dry;
f) removing the formwork (3) and the at least one support frame (4); and
g) initiating a controlled cracking in each desired cracking plane of the sample.

2. - A method according to claim 1, **characterized in that** it further comprises an additional step, before step c), of placing at least one vibrating tool (12) at the bottom of the formwork (3) and step d) of homogenizing the concrete consists of vibrating the concrete using the at least one vibrating tool (12).

3. - A method according to claim 1 or claim 2, **characterized in that** the process further comprises an additional step, between steps d) and e), of surfacing and finishing the concrete at the open upper face (3c).

4. - A method according to any one of claims 1 to 3, **characterized in that** it further comprises, between steps b) and c), the following additional steps:
- making at least one set of primary apertures (5b) in the at least one mesh (5), the apertures (5b) of a set of primary apertures (5b) being aligned along the selected direction normal to the at least one mesh (5) when there is more than one mesh;
- passing a horizontal reinforcement (6) through each set of primary openings (5b);
- fixing the at least one horizontal reinforcement (6) using at least one transverse reinforcement (7); and
- arranging at least one anti-adhesion element (8) on the at least one horizontal reinforcement (6) at an intersection between the at least one horizontal reinforcement (6) and a plane of a mesh (5).

5. - A method according to any one of claims 1 to 4, **characterized in that** it further comprises, between steps b) and c), the following additional steps:
- making at least one secondary opening (5c) in at least one mesh (5);
- positioning a cast-in-place fastener (9) to be tested in each secondary opening (5c);
- fixing each cast-in-place fastener (9) to be tested to the support frame (4) to which is fixed the mesh (5) in which the respective secondary opening (5c) is formed; and the method further comprising, following step e), an additional step of releasing each cast-in-place fastener (9) to be tested from the respective support frame (4).

6. - A method according to claim 5, **characterized in that** the secondary openings (5c) are provided, and the cast-in-place fasteners (9) to be tested are positioned close to the open upper face (3c).

7. - A method according to any one of claims 4 to 6, **characterized in that** it further comprises, before step c), an additional step of placing retaining wires (10) in each opening (5b, 5c) provided in the at least one mesh (5) so as to prevent deformation of the at least one mesh (5) at an opening (5b, 5c) during step c) of pouring the concrete.

8. - A method according to any one of claims 1 to 7, **characterized in that** it further comprises, following step g), the following additional steps:
- making at least one drilling (14) in at least one outer face (2a) of the concrete sample (2), each drilling (14) being provided perpendicular to the corresponding outer face (2a) and in a desired cracking plane; and
- implanting in each drilling (14) an inserted fastener (9) to be tested.

9. - A method according to claim 8, **characterized in that** the drillings (14) are provided, and the inserted fasteners (9) to be tested are implanted, in external faces (2a) of the sample (2) which are vertical at least up to step e) .

10. - A method according to any one of claims 1 to 9, **characterized in that**, in the final state, at least one mesh (5) extends in the sample (2) over the entire corresponding desired cracking plane on the entire depth (L1) of the sample (2), that is, outer dimensions of the mesh (5) are equal to outer dimensions of the sample (2) when viewed in cross-section according to said desired cracking plane.

11. - A method according to claim 5 or 8, or any one of claims 6, 7 or 9, when it depends on claim 5 or 8, **characterized in that**, in the final state, at least one mesh (5) extends from an outer surface (2a) of the sample (2) towards a core thereof by a distance equal to an anchoring depth (L2) of a fastener (9) arranged in the plane of said mesh (5).

12. - A method according to any one of claims 1 to 11, **characterized in that** the initiation of cracking in step g) comprises, for at least one desired cracking plane, the use of expansion means (15) configured to apply a force between parts of the sample (2) located on either side of said desired cracking plane, so as to place the sample (2) in tension.

13. - A method as claimed in claim 12, **characterized in that** it further comprises a final step of using the expansion means (15) to control the size of an opening (Lf) of at least one crack.

14. - A method according to any one of claims 1 to 13, **characterized in that**, in the final state, the crack opening (Lf) is in a range from 0 to 1 mm, preferably in a range from 0.1 to 0.8 mm.

15. - A method according to any one of claims 1 to 14, **characterized in that** the at least one mesh (5) is a fibre mesh, preferably of glass fibres.

16. - A device (1) for producing a cracked concrete sample (2), comprising a formwork (3) comprising a horizontal base (3b) and at least three vertical side panels (3a) to form the formwork (3) with an open upper face (3c), **characterized in that** it further comprises at least one support frame (4) configured to be arranged vertically, transverse to the formwork (3) and to tension a mesh (5) thereon, so as to be configured to carry out a method according to any one of claims 1 to 15.
